(19) Europäisches Patentamt
European Patent Office
Office européen des brevets

(11) Publication number: **0 123 546 A2**

(12) # EUROPEAN PATENT APPLICATION

(21) Application number: 84302729.3

(22) Date of filing: 24.04.84

(51) Int. Cl.$^3$: **A 61 K 31/34**

(30) Priority: 21.04.83 US 487066
13.04.84 US 598510

(43) Date of publication of application:
31.10.84 Bulletin 84/44

(84) Designated Contracting States:
AT BE CH DE FR GB IT LI LU NL SE

(71) Applicant: Norwich Eaton Pharmaceuticals, Inc.
17 Eaton Avenue
Norwich New York 13815(US)

(72) Inventor: Brooks, Robert Raymond
Plymouth Road
Norwich New York 13815(US)

(72) Inventor: Pong, Schwe Fang
Heady's Corners
Norwich New York 13815(US)

(74) Representative: Brooks, Maxim Courtney et al,
Procter & Gamble (NTC) Limited Whitley Road
Longbenton
Newcastle-upon-Tyne NE12 9TS(GB)

(54) **Compositions for treating inflammation and eliciting analgesia.**

(57) Pharmaceutical dosage compositions and methods of use employing 5-(4-chlorophenyl)-2-furancarboxylic acid are disclosed. These compositions and methods are particularly useful in treating inflammation and eliciting analgesia. Pharmaceutical unit-dosage compositions are also disclosed.

EP 0 123 546 A2

# COMPOSITIONS FOR TREATING INFLAMMATION AND ELICITING ANALGESIA

This application is a continuation-in-part of co-pending U.S. Patent Application Serial Number 487,066, filed April 21, 1983.

This invention is concerned with pharmaceutical dosage compositions and methods employing 5-(4-chlorophenyl)-2-furan-carboxylic acid (I). More particularly, this invention is concerned with the treatment of inflammation and the elicitation of analgesia through the use of 5-(4-chlorophenyl)-2-furancarboxylic acid (I).

The compound 5-(4-chlorophenyl)-2-furancarboxylic acid (I)

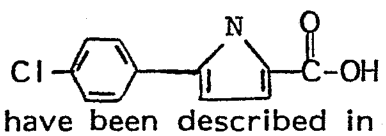 (I)

and its preparation have been described in the chemical literature (Mathur et al., J. Chem. Soc., 1961 (Part 2), 2576-2578). It has now been discovered that this compound exerts impressive anti-inflammatory activity using the well recognized carrageenin-induced rat paw edema method described by Winter et al. in Proc. Soc. Exp. Biol. Med., 111:544 (1962) which finds wide application and acceptance in detecting substances possessing anti-inflammatory activity. Positive results secured under this method are correlative to successful performance in other species where the need for anti-inflammatory medication is present.

## EXAMPLE I

In accordance with the aforementioned method, fasted male Wistar rats, 100-125 g., were dosed in groups of 3 with 3-30 mg/kg p.o. of (I) in 0.5% (w/v) methylcellulose. A control group of 20 rats (groups of 5 rats each combined from 4 separate experiments) received methylcellulose only at the same dose rate of 10 ml/kg. One hour later 0.05 ml of 1% (w/v) carrageenin was injected into the plantar region of the right hindpaw of each rat, using a 27-gauge 1/2-inch needle. Before oral dosing and at 4 and 6 hours post-carrageenin, hindpaw volumes were determined by immersing the feet in a liquid mercury bath to the level of the

anatomical hairline. Displacement of the mercury was determined as a pressure difference, measured through a Statham venous pressure transducer coupled to a Grass Model 7 polygraph. The polygraph was calibrated such that a 1 ml displacement produced a 1 mm pen deflection. Two consecutive measurements were taken in all cases and the mean value used in calculations. Calculation of the $ED_{50}$ value was done as described below for the mouse writhing data. Mean percent protection increased at 4 hours from 0 to 3 mg/kg p.o. to 51.5 at 100 mg/kg p.o. and at 6 hours from 16.6 at 3 mg/kg to 39.9 at 100 mg/kg in a dose-dependent manner. The high dose of 300 mg/kg p.o. gave 34.9 and 23.2% protection at 4 and 6 hours, respectively. The 4 hour $ED_{50}$ value was estimated to be 84.2 mg/kg p.o. In this same test aspirin's $ED_{50}$ value was estimated as 155 mg/kg p.o. (Goldenberg et al., Arch. Int. Pharmacodyn. Ther., 243:331-350, 1980).

EXAMPLE II

The anti-inflammatory activity of (I) was also demonstrated in the standard ultraviolet light-induced cutaneous erythema test in guinea pigs, described by Winder et al. (Arch. Int. Pharmacodyn. Ther., 116:261-292, 1958). Guinea pigs weighing 280-715 g were fasted 24 hours and the dorsal-lateral surface was cleared by clipping and chemical depilation. The animals were placed in an opaque rubber glove with three circular, 6 mm punched holes. Exposure for 90 seconds at a distance of 7 cm to a Model 30620 Hanovia ultraviolet lamp with a Type 616A bulb and Type 6540-001 filter produced the erythema. (I) in methylcellulose was administered orally one hour before irradiation to groups of 5 animals at doses from 5 to 153 mg/kg at a rate of 10 ml/kg. Erythema was scored at 2, 3 and 4 hours post-irradiation as 0 (no redness), 0.5 (slight redness) or 1 (full red circle). Percent protection was calculated with reference to a vehicle-dosed control group of 5 animals treated on the same day. $ED_{50}$ values were estimated by linear regression analysis of the log dose versus percent protection (combined 2, 3 and 4 hour readings) relationship. (I) provided statistically significant dose-dependent protection ranging from 43 to 100% at doses from 15.3 to 153 mg/kg p.o. An

$ED_{50}$ value of 24 mg/kg p.o. was estimated for (I). Aspirin, in a similar evaluation, gave an $ED_{50}$ estimate of 49.5 mg/kg p.o. (Goldenberg et al., op cit.).

EXAMPLE III

The analgesic effect of (I) was demonstrated by its ability to antagonize phenylquinone-induced writhing in mice, as described by Hendershot and Forsaith (J. Pharmacol. Exp. Ther., 125:237-240, 1959). Groups of 10 fasted male albino mice, 14-28 g. were dosed by gavage with 18-1,000 mg/kg (I) in 0.5% (w/v) methylcellulose or with methylcellulose solution alone. One hour later each mouse received 10 ml/kg of 0.02% phenylquinone in 5% ethanol intraperitoneally. The characteristic writhing responses were observed and counted from 5 to 15 minutes after phenylquinone administration. Mean percent protection increased from 13.1 at 18 mg/kg to 92.4 at 1,000 mg/kg in a dose-dependent manner. Linear regression analysis of the relationship between log oral dose and percent protection provided an estimate of 213 mg/kg p.o. for the dose giving 50% protection ($ED_{50}$). In this same test, aspirin's $ED_{50}$ value was estimated at 182 mg/kg p.o.

As used herein, the term "excipient" means an inert carrier or substance (including a gelatin capsule) which aids in the delivery or aesthetic presentation of pharmaceutical dosage forms of (I). This includes compounds which aid in delivery or aesthetic presentation of the acid per os, topically or parenterally.

As used herein, the term "afflicted situs" means the area which is inflamed or which is in need of analgesia.

As used herein, the term "pharmaceutical dosage composition" means a composition comprising safe and effective amounts of (1) a pharmaceutical active and (2) an excipient.

As used herein, the term "safe and effective amount of 5-(4-chlorophenyl)-2-furancarboxylic acid" means a sufficient amount of 5-(4-chlorophenyl)-2-furancarboxylic acid to effectively treat inflammation and/or elicit analgesia at a risk/benefit ratio which is reasonable for such medical treatment. This amount will depend upon factors which include, within the scope of sound medical judgment, the nature and severity of the condition being

treated, the expected necessary duration of treatment, the physical and medical characteristics of the patient, concurrent therapy prescribed, and other factors within the particular knowledge of the patient and physician.

The terms "the acid (I)" and "5-(4-chlorophenyl-2-furancarboxylic acid (I)" are used interchangeably herein.

The pharmaceutical dosage compositions of this invention comprise

(a) a safe and effective amount of 5-(4-chlorophenyl)-2-furancarboxylic acid; and

(b) an excipient.

They may be adapted for oral, topical or parenteral use. However, the preferred adaptation is for administration per os.

Suitable pharmaceutical dosage forms adapted for administration per os of the acid (I) comprise tablets, capsules, powders, granules, elixirs, solutions and suspensions. In all of these dosage forms the acid (I) is used in association with one or more pharmaceutically-acceptable excipients.

The pharmaceutically-acceptable excipients which are employed in association with the acid (I) to form the pharmaceutical dosage form and pharmaceutical unit-dosage form compositions of the present invention are well-known in the art. Their selection inter-alia is predicated (for the most part) upon the selected method of administration. They may be liquids, semi-solids or solids.

The excipients useful in the pharmaceutical dosage compositions of the present invention adapted for oral use include, for example, additives which affect the compressional characteristics of a tablet. These include diluents, binders, adhesives, lubricants, antiadherents and glidants. Excipients useful in the pharmaceutical dosage compositions of the present invention also include non-active ingredients which affect the biopharmaceutics, chemical and physical stability, and overall aesthetics of the final oral dosage form. These include disintegrants, colorants, flavorants, sweeteners, buffers, adsorbents, and the like. For example, excipients useful in oral dosage forms (pharmaceutical

dosage forms adapted for oral administration) include, without limitation, lactose, starch, mannitol, sorbitol, microcrystalline cellulose, dibasic calcium phosphate dihydrate, sucrose-base diluents and binders, sucrose powder, calcium sulfate dihydrate, calcium lactate trihydrate, amdex and cellutab (hydrolyzed starches containing 90-92% dextrose, 3-5% maltose, and higher glucose saccharides to 100%), gums such as tragacanth and acacia, polyvinylpyrrolidone, hydroxymethylcellulose, hydroxypropylcellulose, ethylcellulose, clays, alginates, and pregelatinized corn starches; these compounds are used at their art-established levels.

The pharmaceutical dosage compositions of the present invention adapted for oral use in the form of granules may, for example, comprise the acid (I) in association with an art-disclosed effervescing system, such as sodium bicarbonate, plus a free acid or salt, such as tartaric acid or sodium tartrate.

The pharmaceutical dosage compositions of the present invention adapted for oral use in the form of liquids may be solutions or suspensions. Such compositions may comprise, for example, an aqueous solution of a safe and effective amount of the acid (I) in association with sucrose to provide a syrup. The pharmaceutical dosage compositions of the present invention in the form of suspensions may comprise the acid (I) in association with a solvent plus a suspending agent, flavoring agent, etc.

The pharmaceutical dosage compositions of the present invention which are adapted for topical use may be in the form of an ointment (semi-solid and solid), liquid, paste, cream, gel, or rigid foam. Such compositions comprise the acid (I) in association with topical excipients. Topical excipients useful in the compositions of the present invention include, without limitation, hydrocarbons, silicones, alcohols, polyols and polyglycols, sterols, sterol esters, phenols, carboxylic acids, polyesters, ethers, polyethers, polycarboxylates, polysulfates, polysaccharides, and penetration-enhancing vehicles. Such vehicles are described in detail in U.S. Patent Application Serial No. 516,005, "Penetrating Topical Pharmaceutical Compositions," filed July 20, 1983; U.S.

Patent Application Serial No. 506,275, "Improved Penetrating Topical Pharmaceutical Compositions Containing 1-Dodecyl-Aza-cycloheptan-2-one," filed June 21, 1983; and U.S. Patent Application Serial No. 506,273, "Penetrating Topical Pharmaceutical Compositions Containing N-(2-Hydroxyethyl)Pyrrolidone," filed June 21, 1983; all of which are expressly incorporated herein by reference.

Topical pharmaceutical dosage compositions of the present invention typically contain about 0.5% to about 10%, by weight of the final topical composition, of the acid.

The pharmaceutical dosage compositions of the present invention are preferably administered in unit-dosage form. They may be administered per os, topically or parenterally. Per os administration of solid unit-dosage forms is particularly preferred.

Solid oral unit-dosage forms include tablets, capsules, lozenges, cachets, and pills as well as unit dose powders and granules. Of these, tablets and capsules are preferred. A unit-dose capsule preferably contains from about 10 mg to about 1000 mg of the acid (I) per unit dose. The acid may be in the form of a loose powder, granules, lightly compressed plugs, or even tablets. A unit-dosage capsule more preferably contains about 50 mg to about 800 mg of the acid (I), and most preferably about 200 mg to about 800 mg of the acid (I) per capsule.

A unit-dosage composition containing the acid (I) may be prepared for oral ingestion as a tablet in coated, uncoated, chewable or effervescent form, or in other convenient form. Such tablets contain from about 10 mg to about 1000 mg, preferably about 50 mg to about 800 mg, and more preferably about 200 mg to about 800 mg of the acid (I), per unit-dosage tablet. The unit-dosage forms of the present invention are preferably compressed tablets containing from about 200 mg to about 800 mg of the acid per unit-dosage tablet.

The dosage and/or unit-dosage form compositions of the present invention may contain, in addition to the acid (I), compatible, pharmaceutically-active compounds at their art-disclosed or art-established levels. Such compounds include, without

limitation, other non-steroidal anti-inflammatories, analgesics, and the like. Such anti-inflammatories include, without limitation, the salicylates, acetaminophen, ibuprofen, naproxen, naproxen sodium, indomethacin, ketoprofen, fenoprofen calcium, diflusinal, tolmetin sodium, and the 3-(5-aryl-2-furyl)-3-hydroxypropionic acids, such as 3-[5-(3-chlorophenyl)-2-furyl]-3-hydroxypropionic acid. Unit dosage forms may also contain art-disclosed or art-established levels of other actives frequently used in combination with non-steroidals, such as codeine or caffeine.

This invention further includes: compositions comprising a safe and effective amount of 5-(4-chlorophenyl)-2-furancarboxylic acid and an excipient for treating inflammation; compositions comprising a safe and effective amount of 5-(4-chlorophenyl)-2-furancarboxylic acid and an excipient for eliciting analgesia; and compositions comprising a safe and effective amount of 5-(4-chlorophenyl)-2-furancarboxylic acid and an excipient for treating arthritis.

The present invention still further comprises a method of treating inflammation, or eliciting analgesia, comprising administering to a human or lower warm-blooded animal in need thereof a safe and effective amount of 5-(4-chlorophenyl)-2-furancarboxylic acid. Such administration may be per os, topical or parenteral. Administration per os is preferred.

Optimum dosage levels employed in the methods of present invention will vary according to the type and severity of the condition treated, as well as the method of administration. In general, however, safe and effective oral dosage levels of about 10 mg to about 3,200 mg of the acid (I) per day are useful in the treatment of the pain and inflammation associated with most abnormal and diseased inflammatory states, e.g., arthritis. Oral dosage levels of from about 200 mg to about 3,200 mg per day are preferred, while oral dosage levels of about 400 mg to about 2,400 mg per day are particularly preferred.

Topical treatment regimens according to the practice of this invention comprise applying the compositions herein directly to the skin at the afflicted situs, i.e., the site where the

inflammation exists, or where analgesia is desired. The rate of application and duration of treatment will, of course, depend on many factors. A typical safe and effective usage rate for topical localized treatment is about 1 mg of the total composition per $cm^2$ of skin to about 10 mg of the composition per $cm^2$ of skin. Pharmaceutical dosage compositions of the present invention adapted for topical application within the methods of the present invention are typically applied from about once to about six times daily to the afflicted situs.

EXAMPLE IV

A thorough and intimate mixture of equal parts of 5-(4-chlorophenyl)-2-furancarboxylic acid and a tablet base (cornstarch + 1% magnesium stearate) is prepared. The mixture is compressed into tablets containing 200 mg of 5-(4-chlorophenyl)-2-furancarboxylic acid.

Similar tablets are also prepared containing 400 mg of 5-(4-chlorophenyl)-2-furancarboxylic acid.

EXAMPLE V

A batch of capsules is prepared containing 400 mg of 5-(4-chlorophenyl)-2-furancarboxylic acid each. A second batch is prepared containing 800 mg of 5-(4-chlorophenyl)-2-furancarboxylic acid.

EXAMPLE VI

A composition adapted for topical administration is prepared by melting 50 g polyethylene glycol 4000. An equal amount of polyethylene glycol 400 is added to the melt. The mixture is cooled until congealed. 50 g of 5-(4-chlorophenyl)-2-furancarboxylic acid is then added as a fine powder. The mixture is stirred until the acid is uniformly dispersed.

The resulting composition is applied topically qid (at a rate of 1.5 g/$cm^2$ skin) to tissue which suffers from localized inflammation. A reduction in the inflammation is noted after 72 hours.

EXAMPLE VII

The 200 mg tablets of EXAMPLE IV are administered qid to a human suffering from arthritis. After 2 weeks, a significant reduction in both inflammation and pain is noted. Similar results

are observed when the 400 mg tablets of EXAMPLE IV are administered qid.

EXAMPLE VIII

The 400 mg capsules of EXAMPLE V are administered qid to a human suffering from arthritis. After 2 weeks, a significant reduction in both inflammation and pain is noted. Similar results are observed when the 800 mg capsules of EXAMPLE V are administered qid.

CLAIMS:

1. A therapeutic composition in pharmaceutical dosage form comprising

(a) a safe and effective amount of 5-(4-chlorophenyl)-2-furancarboxylic acid; and

(b) an excipient.

2. A composition according to Claim 1 adapted for oral administration.

3. A composition according to Claim 1 adapted for topical administration

4. A composition according to Claim 2 wherein said composition is in unit-dosage form.

5. A composition according to Claim 4 wherein (a) is present at a level of about 10 mg to about 1000 mg per unit dose.

6. A composition according to Claim 5 wherein (a) is present at a level of about 50 mg to about 800 mg per unit dose.

7. A composition according to Claim 6 wherein (a) is present at a level of about 200 mg to about 800 mg per unit dose.

8. A composition according to Claim 7 wherein said composition is in the form of a tablet or capsule.

MPB/jmc(NA:467R EPO)